# FASCICULE DE BREVET EUROPEEN

(11) **EP 1 571 971 B1**
(45) Date de publication et mention de la délivrance du brevet: **06.12.2006**
(21) Numéro de dépôt: 03773801.0
(22) Date de dépôt: 22.09.2003
(51) Int. Cl.: A61B 1/00, A61B 17/58

(54) **DISPOSITF DE SOLIDARISATION D'UNE PIECE SUR UN SUPPORT**
VORRICHTUNG ZUR VERBINDUNG EINES TEILES AUF EINEN TRÄGER
DEVICE FOR SOLIDLY CONNECTING A PART TO A SUPPORT

(30) Priorité: 24.09.2002 FR 0211802
(43) Date de publication de la demande: 14.09.2005
(73) Titulaire: Stryker Trauma SA, 2545 Selzach (CH)
(72) Inventeur: PORCHER, Robert, F-49360 Yzernay (FR)
(74) Mandataire: Liebetanz, Michael
(86) Numéro de dépôt international: PCT/FR2003/002779
(87) Numéro de publication internationale: WO 2004/028334

(56) Documents cités:
- DE-A- 10 039 767
- FR-A- 2 790 198
- US-A- 5 531 554
- US-A1- 2001 037 112

## Description

La présente invention concerne un dispositif de solidarisation d'une pièce, telle qu'une plaque d'ostéosynthèse, sur un support, tel qu'une masse osseuse, à l'aide d'au moins un organe de fixation affectant la forme d'une tige filetée traversant un orifice logeant une bague de ladite pièce pour venir se visser dans la matière du support.

Les systèmes d'ostéosynthèse par vis et plaque doivent permettre d'immobiliser un ou plusieurs fragments osseux les uns par rapport aux autres. Il est connu d'utiliser des vis avec une tête à embase sphérique coopérant avec un logement sphérique ménagé dans une plaque et d'amener la plaque en compression sur l'os jusqu'à ce que la friction de la plaque sur l'os stabilise l'ensemble. Ces montages permettent de choisir l'angle d'implantation des vis pendant l'intervention et d'opérer un rappel et une compression d'un fragment osseux isolé. Certains de ces systèmes permettent, par la forme oblongue du logement réalisé dans la plaque, d'opérer une compression d'un fragment osseux sur un autre. La faiblesse de ces systèmes est leur faible résistance à des efforts de compression exercés parallèlement au plan de la plaque.

Il est aussi connu d'utiliser une deuxième génération de systèmes vis et plaque dits verrouillés monoaxiaux et verrouillés polyaxiaux dans lesquels la résistance du montage ne repose plus sur une compression de la plaque sur l'os mais sur une fixation de la vis dans la plaque. Ces systèmes permettent d'obtenir un montage distant de l'os avec, pour les plus élaborés, la possibilité de choisir l'angle d'implantation des vis pendant l'intervention tout en obtenant une résistance suffisante aux contraintes postopératoires.

Un exemple de dispositif verrouillé monoaxial est décrit dans le brevet FR-A-2.739.151. Un tel dispositif est constitué d'une plaque traversée de trous taraudés coniques dans lesquels viennent se coincer les têtes filetées coniques des vis en fin de vissage. Un tel système qui obtient un verrouillage par coincement d'un filetage conique dans un trou taraudé conique présente comme inconvénient le fait que comme la vis est engagée à la fois dans la plaque et dans l'os, la distance éventuelle entre plaque et os ne peut plus être réduite. Le rappel d'un fragment osseux est donc impossible. Il en résulte que la distance entre la plaque et l'os doit être réglée avant la mise en place de la vis, ce qui est parfois difficile.

Dans les dispositifs monoaxiaux existants, l'ancrage osseux est parfois amélioré par l'introduction des vis dans des axes non parallèles entre eux, mais au contraire à allure divergente de telle sorte qu'une traction normale à la plaque ne génère aucun arrachement de cette dernière. Pour obtenir une telle orientation des vis, on peut ménager, dans la plaque devant être fixée au support, des orifices d'axe non orthogonal à la surface de la plaque de telle sorte que l'orientation de la vis est fournie par l'orifice ménagé dans ladite plaque. Le chirurgien ne dispose alors d'aucune liberté concernant l'orientation de la vis à fixer. Or, cette orientation prédéterminée peut parfois s'avérer incompatible avec des contraintes anatomiques. Par ailleurs, les dispositifs existants ne permettent pas un rapprochement optimal de la plaque et du support sans nuire à la coaxialité entre axe de la plaque et axe du taraudage.

Un exemple d'un dispositif verrouillé polyaxial est notamment décrit dans le brevet FR-A-2.790.198. Un tel dispositif est constitué d'une plaque équipée d'une bague s'expansant radialement au cours du vissage de la vis de manière à permettre une immobilisation de la bague et par suite de la vis à l'intérieur de l'orifice de la plaque. L'expansion de la bague est obtenue par un filetage conique de la vis et de l'alésage intérieur de la bague.

Il est également connu à travers le brevet DE 100 39 767 un système comprenant une bague de forme extérieure sphérique pouvant s'orienter à l'intérieur d'une plaque comprenant un logement sphérique. La bague est traversée par une vis, dont la tête vient se loger dans un alésage de la bague. Aucun système de blocage de la vis n'est décrit.

Le brevet US-A1-2001/037112 décrit quant à lui un système comprenant une bague de forme extérieure sphérique pouvant s'orienter à l'intérieur d'une plaque comprenant un logement sphérique. La bague est traversée par une vis, dont la tête vient se loger dans un alésage cylindrique de la bague. Le blocage est obtenu par expansion de la tête de la vis au moyen d'une vis conique supplémentaire.

Enfin, le brevet US-A-5.531.554 décrit un système comprenant une vis à collerette déformable. La collerette est restreinte lors de son enfoncement dans un trou conique de la plaque jusqu'à s'étendre au-delà dudit trou, puis s'expanse par élasticité pour empêcher la vis d'être démontée. Le retour de la collerette de la vis à sa forme initiale empêche ainsi un déplacement axial de la vis dans le sens d'une extraction de la vis de l'alésage de la plaque.

Il est à noter que d'autres systèmes obtiennent un verrouillage par introduction en force de la tête filetée de la vis dans le logement de la plaque. La force nécessaire à la déformation de la matière dans le logement est importante. En outre, des risques de détachement de particules métalliques ne peuvent être exclus.

Un but de la présente invention est donc de proposer un dispositif de solidarisation d'une pièce sur un support dont la conception permet un rapprochement optimal de la plaque et du support, une orientation per-opératoire de la vis et une mise en place et un démontage aisés de l'ensemble tout en offrant une bonne résistance à l'arrachement et aux contraintes exercées parallèlement au plan de la plaque.

A cet effet, l'invention a pour objet un dispositif de solidarisation d'une pièce, telle qu'une plaque d'ostéosynthèse, sur un support, tel qu'une masse osseuse, à l'aide d'au moins un organe de fixation affectant la forme d'une tige filetée traversant un orifice logeant une bague de ladite pièce pour venir se visser dans la matière du support, caractérisé en ce que la bague est une bague constrictive dont le profil externe non circulaire coopère avec le profil interne non circulaire de l'orifice de la pièce pour provoquer, lors de l'entraînement en rotation de la bague dans l'orifice, parallèlement à l'immobilisation par coincement de la bague dans l'orifice, une constriction de la bague assurant l'immobilisation de la tige filetée.

Un tel dispositif permet une mise en place aisée de la vis qui n'est pas, avant constriction de la bague, en prise avec la bague. En outre, le rapprochement de la pièce de son support se fait simplement par vissage, la distance entre plaque et support pouvant être réglée à volonté. Enfin, le blocage s'effectue au moyen d'un simple outil qui assure l'entraînement en rotation de la bague.

Le dispositif selon l'invention permet d'utiliser un procédé de pose d'un dispositif de solidarisation d'une pièce, telle qu'une plaque d'ostéosynthèse, sur un support, tel qu'une masse osseuse, à l'aide d'au moins un organe de fixation affectant la forme d'une tige filetée traversant un orifice logeant une bague de ladite pièce pour venir se visser dans ta matière du support, où le procédé consiste à introduire la tige filetée dans la bague elle-même disposée dans l'orifice de la pièce, à visser la tige filetée jusqu'à sa position de vissage final puis à entraîner en rotation la bague de manière à assurer par constriction de la bague l'immobilisation de la tige filetée dans ledit orifice.

L'invention sera bien comprise à la lecture de la description suivante d'exemples de réalisation, en référence aux dessins annexés dans lesquels :
la figure 1 représente une vue de face partiellement en coupe d'un dispositif objet de l'invention à l'état assemblé des éléments le constituant ;
la figure 2 représente une vue de dessus de la bague dans son orifice en absence de toute constriction de la bague sur la vis ;
la figure 3 représente une vue de dessus de la bague dans son orifice en position de constriction de la bague ;

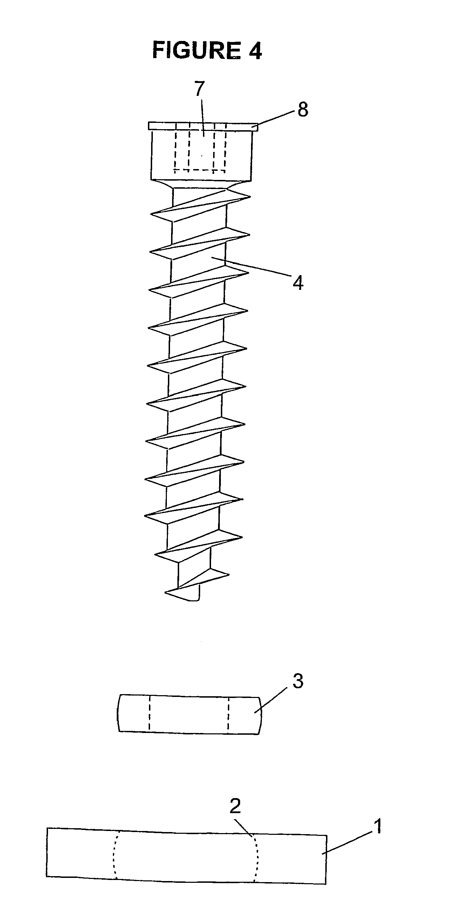

la figure 4 représente une vue de face partiellement en coupe du dispositif de la figure 1 à l'état non assemblé des éléments le constituant et
la figure 5 représente une vue de face partiellement en coupe d'un autre mode de réalisation d'un dispositif conforme à l'invention à l'état assemblé des éléments le constituant.

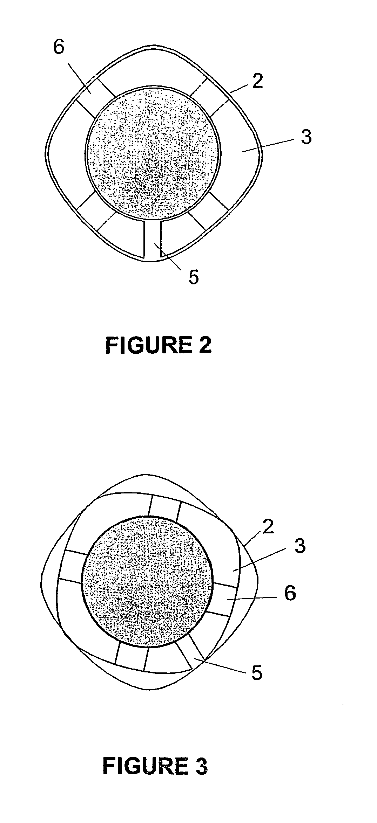

De manière en soi connue, le dispositif, objet de l'invention, est constitué d'une pièce 1 apte à être solidarisée sur un support S au moyen d'au moins un organe 4 de fixation. Comme mentionné ci-dessus, ce dispositif est plus particulièrement destiné au domaine médical. Dans ce cas, la pièce 1 est un implant affectant généralement la forme d'une plaque et le support S au moins un os. L'organe 4 de fixation est quant à lui constitué par une tige filetée sur au moins une partie de sa longueur pouvant encore être appelée vis. Cette tige 4, qui assure la solidarisation de la pièce 1 sur le support S, traverse un orifice 2 de la pièce et vient s'ancrer sur le support S. L'orifice 2 de cette pièce 1 est muni d'une bague 3 traversée par l'organe 4 de fixation. De manière caractéristique à l'invention, cette bague est une bague 3 constrictive lors de son entraînement en rotation dans l'orifice 2, cette constriction de la bague 3 assurant, généralement par coopération avec la tête ou portion d'extrémité de vissage de la tige, une immobilisation de la tige 4 filetée parallèlement à une immobilisation par coincement de la bague 3 dans l'orifice 2. Cette immobilisation par coincement est obtenue par friction de la surface externe de la bague 3 contre la surface interne de l'orifice 2. En effet, le profil externe non circulaire de la bague 3 coopère avec le profil interne non circulaire de l'orifice 2 de la pièce 1 pour provoquer, parallèlement à la constriction de la bague, un coincement de la bague 3 dans l'orifice 2. Il est à noter que le terme circulaire doit être pris dans son sens le plus strict, c'est-à-dire dont tous les points sont situés à égale distance d'un point fixe. Un exemple de réalisation possible d'un tel profil est notamment fourni aux figures 2 et 3. Des profils plus proches de profil circulaire peuvent également être envisagés. Ainsi, un profil constitué de deux départs spiralés successifs peut être envisagé pour le profil extérieur de la bague et le profil intérieur de l'orifice 2. Ces profils peuvent être de forme identique ou non. Pour faciliter la constriction ou resserrement circulaire de la bague 3 autour de la tige 4, cette bague 3 est fendue. La fente est plus particulièrement représentée en 5 aux figures 2 et 3. La constriction aurait pu également être obtenue par déformation de zones déformables à l'intérieur du corps de ladite bague. La mise en place d'une fente constitue toutefois la solution la plus simple pour obtenir une constriction, c'est-à-dire un resserrement circulaire de la bague autour de la tige 4 filetée. La constriction s'opère de préférence par resserrement de la bague autour de la tête ou de la portion d'extrémité de la tige 4 filetée qui est généralement non filetée. Cette tête lisse peut avoir une forme quelconque.

Deux modes de réalisation du dispositif peuvent être envisagés.

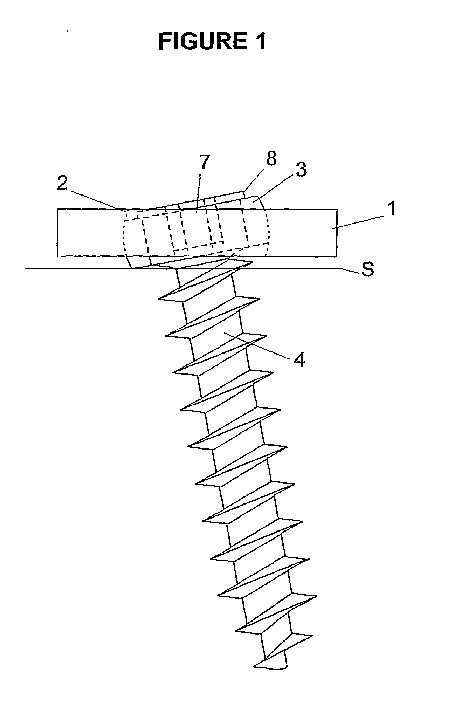

Dans un premier mode de réalisation de l'invention conforme à la figure 1, la bague 3 est montée orientable à l'intérieur de l'orifice 2 de la pièce 1. Ainsi, la bague 3 affecte la forme d'un anneau d'allure générale sphérique fendu se logeant à l'intérieur d'un alésage d'allure générale sphérique de forme complémentaire de l'orifice 2 de la pièce 1. La sphéricité permet d'obtenir une orientation quelconque de la vis à l'intérieur de l'orifice, la bague constituant l'équivalent d'une rotule.

La face frontale de la bague 3 est quant à elle pourvue d'éléments en relief ou en saillie, tels que des rainures 6, adaptés à coopérer avec un outil d'entraînement en rotation de la bague 3. Cette bague 3 peut être pré-montée dans l'orifice 2 de la pièce 1, ce pré-montage s'effectuant en usine. Cette bague 3 peut encore être montée de manière amovible dans l'orifice 2 de la pièce 1. La tige 4 filetée comporte quant à elle, à son extrémité libre dite de vissage, au moins une empreinte 7 en creux ou en relief destinée à coopérer avec un outil de vissage. Dans l'exemple représenté, cette tige 4 est munie, à son extrémité de vissage, d'une cavité axiale prismatique apte à recevoir la tête d'un outil d'entraînement en rotation de la tige. La tête de cette tige filetée est munie, à son extrémité libre de vissage ou au voisinage de cette dernière, d'un épaulement 8 prenant éventuellement appui en position de fin de vissage sur la face frontale de la bague 3 pour permettre un rapprochement de la pièce 1 de son support S. Cet épaulement est plus particulièrement représenté à la figure 1. L'un des avantages de ce dispositif est de pouvoir faire ainsi varier à volonté la distance entre pièce et support bien que le verrouillage de la tige filetée soit garanti indépendamment de la nature et de la qualité de l'interface entre pièce et support.

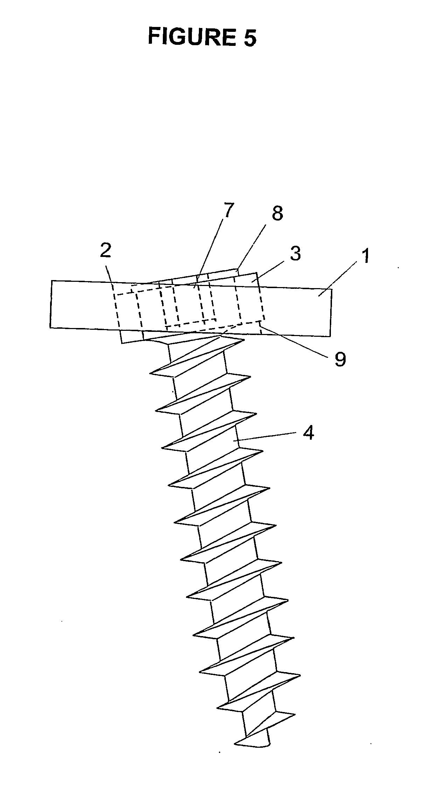

Dans un autre mode de réalisation de l'invention représenté à la figure 5, la pièce 1 est munie d'un orifice pré-orienté muni d'un épaulement 9 interne de retenue de la bague 3 à l'intérieur de l'orifice. Cet épaulement, ménagé dans le fond de l'alésage de la pièce 1 en forme de plaque, retient la bague 3 lors de la phase de compression de la plaque sur l'os ou lors d'un rappel du fragment osseux.

Le procédé de pose d'un tel dispositif est le suivant. Il consiste à introduire la tige 4 filetée dans la bague 3, elle-même disposée dans l'orifice 2 de la pièce 1, à visser la tige 4 filetée jusqu'à sa position de vissage final puis à entraîner en rotation la bague 3 de manière à assurer, par constriction de la bague 3, l'immobilisation de la vis dans l'orifice 2.

De manière plus détaillée, les phases de la mise en place de la tige 4 sont généralement les suivantes. Le chirurgien pratique dans un premier temps un formage de la plaque avec des pinces spéciales préservant l'intégrité des alésages dans la plaque. La plaque est ensuite mise en position sur l'os. Un guide de perçage est introduit dans la bague logée à l'intérieur de la plaque de manière à permettre une orientation de la bague en fonction de l'orientation que le chirurgien souhaite donner à la vis. Le chirurgien procède alors au perçage à travers le guide de perçage qui permet de positionner le trou de la tige filetée dans l'axe de la bague de telle sorte que, axe de tige et axe de bague sont rigoureusement coïncidents. La longueur percée est mesurée au moyen d'une jauge. Une tige filetée de longueur adaptée est alors choisie en fonction de la longueur mesurée par la jauge. La vis est mise en place par entraînement en rotation au moyen d'un tournevis. Ce vissage s'opère jusqu'à rapprochement éventuel de la plaque vers l'os ou rappel d'un segment osseux en continuant à visser alors que l'épaulement de la tête de vis est en contact avec la face frontale de la bague. La plaque comportant généralement plusieurs orifices, l'ensemble des vis est mis en place. Une fois que l'ensemble des vis est positionné, il peut alors être procédé au verrouillage de toutes les vis par entraînement en rotation de chaque bague au moyen d'un outil adapté.

Dans le cas d'un tel dispositif de solidarisation, on ne constate aucun dévissage, recul ou pivotement de la vis dans son orifice. La vis est ainsi immobilisée suivant au moins trois axes, à savoir l'axe du recul, l'axe de rotation de la vis sur son axe et l'axe d'immobilisation en rotation autour du centre de la sphère. L'effort radial qui est appliqué, lors de l'entraînement en rotation de la bague entre la paroi externe de la bague et la paroi intérieure de l'orifice 2, s'exerce de préférence en au moins trois points. La constriction est généralement obtenue par resserrement de la fente.

## Revendications

1. Dispositif de solidarisation d'une pièce (1), telle qu'une plaque d'ostéosynthèse, sur un support (S), tel qu'une masse osseuse, à l'aide d'au moins un organe (4) de fixation affectant la forme d'une tige filetée traversant un orifice (2) logeant une bague (3) de ladite pièce (1) pour venir se visser dans la matière du support (S),
**caractérisé en ce que** la bague (3) est une bague constrictive dont le profil externe non circulaire coopère avec le profil interne non circulaire de l'orifice (2) de la pièce (1) pour provoquer, lors de l'entraînement en rotation de la bague (3) dans l'orifice (2), parallèlement à l'immobilisation par coincement de la bague (3) dans l'orifice (2), une constriction de la bague (3) assurant l'immobilisation de la tige (4) filetée.

2. Dispositif selon la revendication 1,
**caractérisé en ce que** la bague (3) est fendue.

3. Dispositif selon l'une des revendications 1 et 2,
**caractérisé en ce que** la bague (3) est montée orientable à l'intérieur de l'orifice (2) de la pièce (1).

4. Dispositif selon l'une des revendications 1 à 3,
**caractérisé en ce que** la bague (3) affecte la forme d'un anneau d'allure générale sphérique fendu se logeant à l'intérieur d'un alésage d'allure générale sphérique de forme complémentaire de l'orifice (2) de la pièce (1).

5. Dispositif selon l'une des revendications 1 à 4,
**caractérisé en ce que** la face frontale de la bague (3) est pourvue d'éléments en relief ou en saillie, tels que des rainures (6), adaptés à coopérer avec un outil d'entraînement en rotation de la bague (3).

6. Dispositif selon l'une des revendications 1 à 5,
**caractérisé en ce que** la bague (3) est pré-montée dans l'orifice (2) de la pièce (1).

7. Dispositif selon l'une des revendications 1 à 5,
**caractérisé en ce que** la bague (3) est montée de manière amovible dans l'orifice (2) de la pièce (1).

8. Dispositif selon l'une des revendications 1 à 7,
**caractérisé en ce que** la tige (4) filetée comporte à son extrémité libre dite de vissage au moins une empreinte (7) en creux ou en relief destinée à coopérer avec un outil de vissage.

9. Dispositif selon l'une des.revendications 1 à 8,
**caractérisé en ce que** la tige (4) filetée est munie, à son extrémité libre dite de vissage, d'un épaulement (8) prenant éventuellement appui, en position de fin de vissage, sur la face frontale de la bague (3), pour permettre un rapprochement de la pièce (1) de son support (S).

10. Dispositif selon l'une des revendications 1 et 2,
**caractérisé en ce que** la pièce (1) est munie d'un orifice (2) pré-orienté muni d'un épaulement (9) interne de retenue de la bague (3) à l'intérieur de l'orifice (2) de la pièce (1).

## Claims

1. A device for solidly connecting a part (1), such as an osteosynthesis plate, on a support (S), such as a bone mass, comprising at least one fixing element (4) in the form of a threaded rod passing through a hole (2) housing a ring (3) in the said part (1), to be screwed into the material of the support (S), **characterized in that** the ring (3) is a constrictive ring having a non-circular external profile cooperating with a non-circular internal profile of the hole (2) of the part (1) to cause, during rotational movement of the ring (3) in the hole (2), at the same time an immobilization by wedging of the ring (3) in the hole (2) as well as a constriction of the ring (3) ensuring the immobilization of the threaded rod (4).

2. The device according to claim 1, wherein the ring (3) is slotted.

3. The device according to any of claims 1 or 2, wherein the ring (3) is mounted inside the hole (2) of the part (1) in a orientable manner.

4. The device according to any of claims 1 to 3, wherein the ring takes the form of a slotted ring (3) having a generally spherical outer surface being housed inside a generally spherical bore having a complementary form to said hole (2) of part (1).

5. The device according to any of claims 1 to 4, wherein the front face of the ring (3) is fitted with embossed or hollowed coupling elements such as flutes (6), shaped to cooperate with a rotational drive tool for the ring (3).

6. The device according to any of claims 1 to 5, wherein the ring (3) is premounted in the hole (2) of the part (1).

7. The device according to any of claims 1 to 5, wherein said ring (3) is removably mounted in the hole (2) of the part (1).

8. The device according to any of claims 1 to 7, wherein the threaded rod (4) has on its free end for screwing at least one embossed or hollowed recess (7) intended to cooperate with a driving tool.

9. The device according to any of claims 1 to 8, wherein the threaded rod (4) is provided, on its free end for screwing, with a shoulder (8), being engagable with the front face of the ring (3) at the position at the end of screwing, to allow a mating of the part (1) with its support (5).

10. The device according to claim 1 or 2, wherein the part (1) is provided with a pre-oriented hole (2) provided with an internal shoulder (9) for retaining of the ring (3) inside the hole (2) of the part (1).

## Patentansprüche

1. Vorrichtung zur Befestigung eines Teiles (1) wie eine Platte für die Osteosynthese, auf einem Träger (S) wie eine Knochenmasse, mit Hilfe mindestens eines Befestigungselementes (4), welches die Gestalt einer gewindeten Stange aufweist, die eine Öffnung (2) durchquert, welche einen Ring (3) des besagten Teiles (1) aufnimmt, um sich in die Trägermaterie (1) einzuschrauben, **dadurch gekennzeichnet, dass** der Ring (3) ein Einschränkungsring ist, dessen nicht kreisförmiges äusseres Profil mit dem inneren nicht kreisförmigen Profil der Öffnung (2) des Teils (1) zusammenwirkt, um während der Drehung des Ringes (3) in der Bohrung (2) parallel zur Immobilisierung durch Verklemmung des Ringes (3) in der Öffnung (2) eine Verschränkung des Ringes (3) zu bewirken, die eine Immobilisierung des gewindeten Schaftes (4) gewährleistet.

2. Vorrichtung nach Anspruch 1, **dadurch gekennzeichnet, dass** der Ring (3) geschlitzt ist.

3. Vorrichtung nach einem der Ansprüche 1 oder 2, **dadurch gekennzeichnet, dass** der Ring (3) in orientierbarer Weise im Inneren der Öffnung (2) des Teils (1) montiert ist.

4. Vorrichtung nach einem der Ansprüche 1 bis 3, **dadurch gekennzeichnet, dass** der Ring (3) die Gestalt eines geschlitzten Ringes mit einer allgemeinen sphärischen Form annimmt, welcher sich im Inneren einer Bohrung von allgemein sphärischer Form in komplementärer Form zur Öffnung (2) des Teiles (1) einpasst.

5. Vorrichtung nach einem der Ansprüche 1 bis 4, **dadurch gekennzeichnet, dass** die Vorderseite des Ringes (3) mit Elementen überstehend oder ausnehmend versehen ist, wie Rillen (6), die geeignet sind, mit einem Drehwerkzeug für den Ring (3) zusammenzuwirken.

6. Vorrichtung nach einem der Ansprüche 1 bis 5, **dadurch gekennzeichnet, dass** der Ring (3) in der Öffnung (2) des Teiles (1) vormontiert ist.

7. Vorrichtung nach einem der Ansprüche 1 bis 5, **dadurch gekennzeichnet, dass** der Ring (3) in entfernbarer Weise in der Öffnung (2) des Teils (1) angeordnet ist.

8. Vorrichtung nach einem der Ansprüche 1 bis 7, **dadurch gekennzeichnet, dass** der gewindete Schaft (4) an seinem freien Ende der Verschraubung mindestens ein Gepräge (7) ausgenommen oder überstehend aufweist, welches dafür vorgesehen ist, mit einem Schraubwerkzeug zusammenzuwirken.

9. Vorrichtung nach einem der Ansprüche 1 bis 8, **dadurch gekennzeichnet, dass** der gewindete Schaft (4) an seinem freien Ende der Verschraubung mit einer Schulter (8) versehen ist, die eventuell in der Position am Ende der Verschraubung auf der Vorderseite des Ringes (3) Abstützung sucht, um eine Annäherung des Teiles (1) an seine Träger (S) zu gestatten.

10. Vorrichtung nach einem der Ansprüche 1 oder 2, **dadurch gekennzeichnet, dass** das Teil (1) mit einer Öffnung (2) versehen ist, die in vororientierter Weise mit einer inneren Schulter (9) versehen ist, um den Ring (3) im Inneren der Öffnung (2) des Teils (1) zurückzuhalten.
